# EUROPEAN PATENT SPECIFICATION

(11) **EP 2 952 180 B1**
(45) Date of publication and mention of the grant of the patent: **25.01.2017**
(21) Application number: 14001940.7
(22) Date of filing: 04.06.2014
(51) Int. Cl.: A61K 9/16, A61K 9/20, A61K 31/05, A61K 31/202

(54) **Solid formulations containing resveratrol and omega-3 polyunsaturated fatty acids (n-3 PUFA)**
Feste Formulierungen mit Resveratrol und mehrfach ungesättigten Omega-3-Fettsäuren (n-3 PUFA)
Formulations solides contenant du resvératrol et des acides gras polyinsaturés omega -3 (n-3 PUFA)

(43) Date of publication of application: 09.12.2015
(73) Proprietor: SIGMA-TAU Industrie Farmaceutiche Riunite S.p.A., 00144 Rome (IT)
(72) Inventor: Santaniello, Mosé, 00048 Nettuno (RM) (IT); Giannini, Giuseppe, 00040 Pomezia (RM) (IT)
(74) Representative: Hiebl, Inge Elisabeth

(56) References cited:
- WO-A1-2009/034124
- WO-A1-2011/120530
- US-A1- 2010 247 632

## Description

### FIELD OF THE INVENTION

The present invention relates to solid compositions comprising omega-3 polyunsaturated fatty acid (n-3 PUFA) and resveratrol, or a natural extract containing resveratrol, adsorbed on an inert substrate wherein the composition is maintained stable for 6 months at room temperature.

In particular, the present invention relates to the solid and stable compositions mentioned above, for use in the prevention or treatment of cardiovascular diseases due to lipid metabolism disorders and increased platelets aggregation, damages due to free radicals and/or viral diseases.

### BACKGROUND OF THE INVENTION

Cardiovascular diseases related to abnormal lipid metabolism are very frequent in industrialised countries. In Italy, for instance, according to the data from the World Health Organization published on April 2011, the number of deceases in Italy for cardiovascular diseases reached 18.65% of the overall mortality. Our knowledge of the relationships between cholesterol and coronary heart disease stem from epidemiological studies conducted over the past few years. The conclusions reached in these studies indicate that the development of severe coronary atherosclerosis and coronary heart disease are closely correlated with serum cholesterol levels (Breuer, H.W. M.; European Cardiology, 2005; 1-6).

Platelets play an important, but often under-recognized role in cardiovascular disease. For example, the normal response of the platelet can be altered, either by increased pro-aggregatory stimuli or by diminished anti-aggregatory substances to produce conditions of increased platelet activation/aggregation occuring in cardiovascular disease states both on a chronic (e.g. stable angina pectoris) and acute basis (e.g. acute myocardial infarction). In addition, platelet hyperaggregability is also associated with risk factors of coronary artery disease (e.g. smoking, hypertension, and hypercholesterolemia). Finally, the utility of an increasing range of anti-platelet therapies in the management of the above disease states further emphasizes the pivotal role platelets play in the pathogenesis of cardiovascular disease. A recently published paper provides a comprehensive overview of the normal physiologic role of platelets in maintain homeostasis, the pathophysiologic processes that contribute to platelet dysfunction in cardiovascular disease and the associated role and benefits of anti-platelet therapies (Kottke-Marchant K.: Cleveland Clinic Journal of Medicine; 2009 Apr; 76(1): 1-7).

Evidence is accumulating that most of the degenerative diseases that afflict humanity have their origin in deleterious free radical reactions. These diseases include atherosclerosis, cancer, inflammatory joint disease, asthma, diabetes, senile dementia and degenerative eye disease. The process of biological ageing might also have a free radical basis. Most free radical damage to cells involves oxygen free radicals or, more generally, activated oxygen species (AOS) which include non-radical species such as singlet oxygen and hydrogen peroxide as well as free radicals. The AOS can damage genetic material, cause lipid peroxidation in cell membranes, and inactivate membrane-bound enzymes. Antioxidant supplementation of our diet is needed to ensure a more healthy elderly population (Aust. N. Z .J Ophthalmol. 1995 Feb;23(1):3-7).

Omega-3 polyunsaturated fatty acids (n-3 PUFA) have demonstrated a beneficial effect in the prevention of cardiovascular events (Aarsetoey H. et al.;Cardiology Research and Practice, Volume 2012: 1-16), possibly by means of an antiinflammatory, antithrombotic and antiarrhythmic mechanism (Sethi S. et al.; Blood 2002:100:-1340-6; Billman GE, et al.; Circulation 3 1999: 99:2452-7). The hypolipidic effect was the first detected, so at first these drugs had been used for the treatment of dislipidemic disorders, while the antiinflammatory, antithrombotic, antiatherosclerotic and antiarrhythmogenic effects have been found later. GISSI-Prevention trial (Lancet 1999 354: 447-55) was the first trial demonstrating the efficacy and tolerability of n-3 PUFAs in post-myocardial infarction patients. According to the evidence in literature, today n-3 PUFAs are indicated for the primary and secondary prevention of ischemic cardiopathy and sudden cardiac death (SCD) (Mori TA, Beilin L J. Long-chain omega-3 fatty acids, blood lipids and cardiovascular risk reduction. Curr. Opin. Lipidol. 2001;12:11-7).

In Nutrition and Dietary Supplements, 2011 September 14;: 93-100 it is described the role of n-3 series polyunsaturated fatty acids in cardiovascular disease prevention. Resveratrol (trans-3,4',5,-trihydroxystilbene) is a polyphenol molecule located in the skins of black grapes. It is known that it has cardioprotective effects, acting as inhibitor of platelet aggregation (Szmitko PE, et al. Circulation January 2005, 111 (2) p10-11; Das DK, et al. "Resveratrol in cardioprotection: a therapeutic promise of alternative medicine". Mol. Interv., 2006, 6 (1): 36-47). It also acts as antioxidant and skin protecting agent (Afaq, Farrukh et al. "Botanical antioxidants in the prevention of photocarcinogenesis and photoaging"; Experimental Dermatology, 2006, 15 (9): 678-84). Resveratrol has been intensively studied recently, in relation to the known beneficial properties of red wine, of which it is one of the fundamental ingredients *(*Life Sci., 71, 2145-52, 2002*).* Numerous studies have demonstrated an anticarcinogenic activity of resveratrol, whose mechanisms of action can be subdivided as follows: inhibition of activation of transcription factor NF-kB, capable of regulating the expression of various genes involved in inflammatory and carcinogenic processes (Lancet, 341, 1103-1104, 1993*;* Science, 275, 218.220, 1997*;* Proc. Natl. Acad. Sc., 94, 14138-14143, 1997*;* Life Science, 61, 2103-2110, 1997*;* Brit. J. Pharm., 126, 673-680, 1999; J. Imm., 164, 6509-6519, 2000*);* inhibition of various proteins, including protein kinase C *(*Biochemistry., 38, 13244-13251, 1999*),* ribonucleotide reductase *(*FEBS Lett., 421, 277-279, 1998*)* and cyclo-oxygenase-2 (COX-2) in mammalian epithelial cells *(*Ann. N.Y. Acad Sci, 889, 214-223, 1999*;* Carcinogenesis., 21, 959-963, 2000*);* activation of caspases 2, 3, 6 and 9 (FASEB J., 1613-1615, 2000*)* and modulation of the gene p53, which is a known tumour suppressor (Cancer Research, 59, 5892-5895, 1999*;* Clin. Biochem., 34, 415-420, 2001*).* In Free Radic. Res., 33, 105-114, 2000 it is described the antioxidant activity of resveratrol and its ability to counteract the damaging effects produced by various substances and/or conditions that cause intracellular oxidative stress.

In EP1567137B1 it is described the use of resveratrol for treating influenza virus infections.

WO2009/03412 discloses a composition comprising a core comprising an oxidation-labile compound and an oxidation protective layer.

US2010/247632 relates to enhancing oral bioavailability for poorly water-soluble beneficial agents.

WO 2011161501 describes solid compositions in form of powders or granulates wherein the active ingredient is insoluble or poorly soluble in water and/or thermolabile and/or having unpleasant organoleptic properties. The method described is a dispersion in a lipid matrix containing a triglyceride, a polyoxyethylene sorbitan ester and ascorbyl palmitate.

Patent application WO 2011120530 discloses solid porous inert carrier compositions comprising a porous silicium dioxide (silicon dioxide) and a release enhancing agent that can be loaded with pharmaceutical oils, among which fish oil. An example of preparation of compositions comprising n-3 PUFA in combination with one or more active ingredients is described in EP2517697. In particular, are described microcapsule suspensions comprising one or more statins in alkyl esters of n-3 PUFA, in which the statins are isolated from contact with the alkyl ester of n-3 PUFA by means of a polymeric membrane that can be easily disintegrated in the gastrointestinal medium.

Most of the methods known in the art, useful for preparing compositions containing resveratrol and n-3 PUFA, include microencapsulation or coating processes which are long and expensive. These studies are focused on finding a suitable delivery method for the of resveratrol in a single dose within an oily phase.

In the art, solid compositions comprising n-3 PUFA and resveratrol, in which n-3 PUFA are stable at room temperature for up to 6 months, are not known.

Considering the advantages of having formulations containing n-3 PUFA in solid form, for the preparation of formulations which could combine multiple nutraceuticals that in most cases are dry extracts or insoluble in n-3 PUFA, it becomes more and more noticeable the need to find a method for preparing compositions in which n-3 PUFA is prevented from degradation through a simple, fast and economically advantageous process.

### DESCRIPTION OF THE INVENTION

It has now surprisingly been found that when the n-3 PUFA are adsorbed on an inert substrate, the addition of a suitable amount of resveratrol, or a natural extract containing resveratrol, provides stability to the formulation for at least 6 months at room temperature.

It is therefore an object of the present invention a solid composition comprising omega-3 polyunsaturated fatty acids (n-3 PUFA) and resveratrol adsorbed on an inert substrate selected from the group consisting of fine ultra-light granule of magnesium aluminometasilicate or a silica carrier, wherein the recovery of n-3 PUFA is at least 96% after 6 months at 25°C at 60% RH; at least 95% after 3 months at 30°C at 60% RH.; and at least 90% after 3 months at 40°C at 70% RH.

In one embodiment of the present invention the omega-3 polyunsaturated fatty acids (n-3 PUFA) are selected from the group consisting of eicosapentaenoic acid (EPA), docosahexaenoic acid (DHA) or their alkyl esters, and mixtures thereof; wherein the alkyl esters are selected from the group consisting of ethyl, methyl, and propyl esters. In a further embodiment of the present invention the omega-3 polyunsaturated fatty acids (n-3 PUFA) are a mixture of fatty acids having a content in EPA and DHA comprised between 75% and 95% by weight, preferably at least 85%, on the total fatty acids weight, and wherein the total content of n-3 PUFA is at least 90% by weight on the total fatty acids weight; and the ratio between eicosapentaenoic acid and docosahexaenoic acid is comprised between 0.5 and 2;

In another embodiment of the present invention the omega-3 polyunsaturated fatty acids (n-3 PUFA) are a mixture of ethyl esters of EPA and DHA in a ratio comprised between 0.9 and 1.5 and the content of EPA ethyl ester is comprised between 40 and 51 % and the content of DHA ethyl ester is comprised between 34 and 45% by weight on the total fatty acids weight;
In another embodiment of the present invention the inert substrate is selected from Neusilin® US2 and SYLOID® XDP.

It is a further object of the present invention a solid composition comprising omega-3 polyunsaturated fatty acids (n-3 PUFA) and resveratrol adsorbed on an inert substrate selected from the group consisting of fine ultra-light granule of magnesium aluminometasilicate or a silica carrier, wherein the recovery of omega-3 is at least 96% after 6 months at 25°C; at least 95% after 3 months at 30°C.; or at least 90% after 3 months at 40°C., in the form of dietary or nutritional supplement, or medicament for oral administration.

In a further embodiment of the present invention in the composition above-mentioned the omega-3 polyunsaturated fatty acids (n-3 PUFA) are in an amount comprised between 0.5 and 1.0 g, preferably between 0.8 and 0.9 g, more preferably 0.9 g;

It is a further object of the present invention the above-mentioned composition, wherein the ratio between omega-3 polyunsaturated fatty acids (n-3 PUFA) and resveratrol is comprised between 0.01 and 0.1, preferably 0.02.

According to a further embodiment of the present invention, the above-mentioned composition, can further comprise one or more vitamins, minerals, coenzymes, antioxidants and/or plant extracts.

According to a further embodiment of the present invention, the above-mentioned composition, further comprises at least one pharmaceutically acceptable vehicle or excipient.

It is a further object of the present invention, the above-mentioned composition, characterized in that it is encapsulated by soft gelatin capsules, optionally having an enteric coating, for oral administration.

It is a further object of the present invention the above-mentioned compositions, for use in preventing or treating cardiovascular diseases due to lipid metabolism disorders and/or increased platelets aggregation; damages due to free radicals selected from the group consisting of atherosclerosis, cancer, inflammatory joint disease, asthma, diabetes, senile dementia and degenerative eye disease; and/or viral diseases.

The pharmaceutical composition suitable for use according to the present invention generally comprises at least one pharmaceutically acceptable vehicle and/or one diluent and/or one surfactant and/or one thickener and/or one binder and/or one lubricant and/or one aromatizer and/or one colorant and/or one stabilizer, which can easily be selected by the expert of the art.

The compositions of the invention, together with a conventionally employed adjuvant, carrier, diluent or excipient may be placed into the form of pharmaceutical solid compositions and unit dosages thereof. Such pharmaceutical compositions may be employed as solids, such as tablets or filled capsules, all for oral use. The compositions for oral administration may also take the form of bulk suspensions or powders. More commonly, however, the compositions are presented in unit dosage forms to facilitate accurate dosing.

The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient.

Further materials as well as processing techniques and the like are set out in Part 5 of Remington's Pharmaceutical Sciences, 20th Edition, 2000, Merck Publishing Company, Easton, Pennsylvania, which is incorporated herein by reference.

The compositions according to the present invention can also be formulated as a food supplement or dietary supplement, which constitute a further object of the invention. The compositions according to the present invention comprise active ingredients which are known in the art and already used in clinical practice. Therefore, they are very easy to procure, inasmuch as they are products which have been on the market for some time and are of a grade suitable for human or animal administration.

Resveratrol (3,5,4'-trihydroxy-trans-stilbene) is a stilbenoid, a type of natural phenol, and phytoalexin produced naturally by several plants when under attack by pathogens such as bacteria or fungi.

The term "omega-3 polyunsaturated fatty acids" (here abbreviated as "n-3 PUFA") relates to a family of long-chain polyunsaturated fatty acids, generally C₁₆-C₂₄, in particular those having a C₂₀-C₂₂ chain. They all have in common a carbon-carbon double bond in the n-3 position, i.e. the third bond from the methyl end of the fatty acid. Examples of the most common omega-3 polyunsaturated fatty acids found in nature are reported in the Table below together with their assigned names.

| Common name | Lipid name | Chemical name |
|---|---|---|
| Roughanic acid | 16:3 (n-3) | all-cis-7,10,13-hexadecatrienoic acid |
| α-Linolenic acid (ALA) | 18:3 (n-3) | all-cis-9,12,15-octadecatrienoic acid |
| Stearidonic acid (STD) | 18:4 (n-3) | all-cis-6,9,12,15-octadecatetraenoic acid |
| Eicosatrienoic acid (ETE) | 20:3 (n-3) | all-cis-11,14,17-eicosatrienoic acid |
| Eicosatetraenoic acid (ETA) | 20:4 (n-3) | all-cis-8,11,14,17-eicosatetraenoic acid |
| Eicosapentaenoic acid (EPA) | 20:5 (n-3) | all-cis-5,8,11,14,17-eicosapentaenoic acid |
| Docosapentaenoic acid (DPA),Clupanodonic acid | 22:5 (n-3) | all-cis-7,10,13,16,19-docosapentaenoic acid |
| Docosahexaenoic acid (DHA) | 22:6 (n-3) | all-cis-4,7,10,13,16,19-docosahexaenoic acid |
| Tetracosapentaenoic acid | 24:5 (n-3) | all-cis-9,12,15,18, 21-Tetracosapentaenoic acid |

The ones most preferred are *all-cis-*5,8,11,14,17-eicosapentaenoic acid (EPA) and *all-*cis-4,7,10,13,16,19--docosahexaenoic acid (DHA).

Preferably the n-3 PUFA according to the invention is a mixture of fatty acids having a high content in EPA and DHA, for example with a content in EPA and DHA higher than 25% by weight, preferably from about 30% to about 100% by weight, in particular about between 75% and 95%, and more preferably at least 85% by weight based on the total fatty acid weight. Preferably the total content of n-3 PUFA according to the invention is a mixture of fatty acids having at least 90% of n-3 PUFA by weight based on the total fatty acid weight.

The terms "PUFA" and "n-3 PUFA", as used here, are intended to encompass their corresponding C₁-C₃ alkyl esters, preferably their ethyl esters, and/or their salts with pharmaceutically acceptable bases such as sodium hydroxide, lysine, arginine or aminoalcohols such as choline. The compositions of the invention are administered orally, in particular in the form of soft gelatin capsules. The unit dose generally comprises 100-1000 mg of polyunsaturated fatty acids of the omega-3 series, preferably 500-1000 mg or 300-500 mg, the total dose being usually around 0.1-3.0 g per day, preferably 0.3-2.0 g per day, most preferably 1.0 g per day.

Specific drugs containing n-3 PUFA that meet the above specifications, as active ingredient and that can be used according to the present invention, are already available on the market.

This amount of product may be administered in the form of several daily divided doses or preferably as a single dose, in order to reach the desired blood level. Of course, the clinician may vary the amount of product (or mixture with another therapeutic agent) to be administered, according to the patient's conditions, age and weight.

Other types of formulation for oral administration are also suitable for the purposes of the invention; for example hard capsules or tablets, in which the polyunsaturated fatty acids are adsorbed on solid supports.

Those compositions illustrated in the European Pharmacopoiea 2000 (EuPh. 2000), containing quantities greater than or equal to 90wt% of omega-3 polyunsaturated fatty acid (n-3 PUFA) polyunsaturated fatty acid ethyl esters, of which an amount greater than or equal to 80wt% is represented by of mixtures of EPA and DHA ethyl esters are also suitable for the purposes of the present invention.

The most preferred ratio between EPA and DHA is about 0.6-1.1/1.3-1.8; in particular is comprised between 0.9 and 1.5.

Preferably the content of EPA (as ethyl ester) is comprised between 40 and 51% by weight and the content of DHA (as ethyl ester) is comprised between 34 and 45% by weight on the total fatty acids weight.

Neusilin® U.S.-2 (chemical Formula Al₂O₃·MgO·1.7SiO₂·xH₂O, CAS Number 12511-31-08) is a fine ultra-light granule of magnesium aluminometasilicate and is accepted as a multifunctional excipient that improves the quality of pharmaceuticals. Due to its large surface area and porous nature, it adsorbs high loads of oils or water and can be mechanically compacted into high quality tablets.

SYLOID® XDP silica carrier is an optimized mesoporous material engineered for transforming liquids to free flowing solids, particularly oily actives and lipid-based systems. The balance of absorption capacity, density, and release can increase API loading and desorption in liquid-solid formulations and simplify the transformation of lipid based drug delivery systems and API's into free flowing powders for solid dosage forms.

In another embodiment of the present invention, the compositions have a unitary form, in which the active ingredients are present in a single pharmaceutical form, particularly adsorbed on an inert support. The compositions according to the present invention optionally contain, along with the active ingredient, at least one pharmaceutically acceptable vehicle or excipient.

In order to demonstrate its substantial advantages and unexpected effects, the present invention is carried out according to following examples, without limiting it.

### DETAILED DESCRIPTION OF THE INVENTION

### EXAMPLES

### Materials and Methods:

For the purpose of the present invention the following materials have been used:
- the omega-3 polyunsaturated fatty acids (n-3 PUFA) are a mixture of ethyl esters of polyunsaturated fatty acids with a content in EPA and DHA greater than 85%, in a ratio EPA/DHA comprised between 0.9 and 1.5, and is a product provided by Pronova, Norway;
- the resveratrol is furnished by Royalmount Pharma, Montreal, Canada;
- the magnesium aluminometasilicate also known under the trade name Neusilin® used is furnished by Fuji Chemical Industry Co., Ltd, Japan;
- the α-tocopherol is furnished by Sigma-Aldrich s.r.l., Milan, Italy;
- the Syloid XDP is furnished by Grace Performance Chemicals, Worms, Germany
- the Coenzyme Q10 is furnished by Sigma-Aldrich s.r.l., Milan, Italy;
- the ascorbyl palmitate is furnished by Sigma-Aldrich s.r.l., Milan, Italy;
- the lipoic acid is furnished by Sigma-Aldrich s.r.l., Milan, Italy;
- the ascorbic acid is furnished by Sigma-Aldrich s.r.l., Milan, Italy;
- the hydroxytyrosol is furnished by Probeltebio Murcia, Spain;
- the epigallocatechin is furnished by Sigma-Aldrich s.r.l., Milan, Italy

### Stability Analysis:

The formulations were divided into 3 groups and left respectively for 3 and 6 months at 25°C at 60% RH (relative humidity), for 3 and 6 months at 30°C at 60% RH or for 3 and 6 months at 40°C at 70% RH.

The HPLC Analysis was the analysis performed in order to evaluate the stability of the formulations. It was performed using a Column Symmetry C-18 4.6x150mm, a solution of CH₃CN/CH3OH/H₂O 45/45/10 as eluent, a flow of 1m1/min and a Mass spectrometry Detector. The percent amount of EPA and DHA detected are expressed as % by weight.

Other stability tests were also performed:
Stability analysis after autoxidation induced by radiation and by high temperature was performed according to the following procedure:
   - Autoxidation induced by radiation: an amount of 100 mg of the formulation is dispersed in a 5 cm diameter Petri dish, which is placed at a distance of 12cm from the light source and exposed to light for 24 hours. The light source is constituted by a 13W lamp with an emission spectrum of from 300 to 640 nm, with peaks at: 440, 490, 540, 590 and 610 nm. The test is carried out at 22°C at 60% RH
   - autoxidation induced by high temperature: an amount of 100 mg of the formulation is placed in an amber container and put in a stove at 60° C for 1 month

### Formulation 1

| | |
|---|---|
| - n-3 PUFA: | 5,0 g |
| - Neusilin U.S.-2: | 5,0 g |

n-3 PUFA were added to Neusilin U.S.-2 in small portions, manually mixing up to obtain a homogeneous solid. Stability tests are carried out at 25°C at 60% RH, 30°C at 60% RH and 40°C at 70% RH as described above for 1, 3 and 6 months.

The results obtained are summarized Table 1

**Table 1**

| Temperature (°C) | 1 month | | 3 months | | 6 months | |
|---|---|---|---|---|---|---|
| | Recovery EPA (%) | Recovery DHA (%) | Recovery EPA (%) | Recovery DHA (%) | Recovery EPA (%) | Recovery DHA(%) |
| 25 | 98 | 97 | 33 | 28 | 0 | 0 |
| 30 | 85 | 86 | 21 | 16 | 0 | 0 |
| 40 | 69 | 67 | 0 | 0 | 0 | 0 |

Other formulations were prepared, adding a natural antioxidant to formulation 1, in order to assess the inhibition of the degradation of omega-3.

The results of the stability tests performed after autoxidation induced by radiation are resumed in Table 2

**Table 2**

| Antioxidant | Temperature (°C) | Time (hours) | % w/w Antioxidan | Recovery EPA (%) | Recovery DHA (%) |
|---|---|---|---|---|---|
| Not used | 22 | 24 | 0 | <5 | <5 |
| Resveratrol | 22 | 24 | 1.14 | 57 | 55 |
| α-tocopherol | 22 | 24 | 2.15 | 51 | 47 |
| Coenzyme Q10 | 22 | 24 | 4.32 | 55 | 53 |
| Ascorbyl palmitate | 22 | 24 | 2.07 | 15 | 13 |
| Epigallocatechin | 22 | 24 | 2.30 | <5 | <5 |
| Hydroxytyrosol | 22 | 24 | 0.77 | <5 | <5 |
| Oleorupine | 22 | 24 | 2,70 | <5 | <5 |
| Ascorbic acid | 22 | 24 | 0.88 | <5 | <5 |
| Lipoic acid | 22 | 24 | 1.00 | <5 | <5 |

The results of the stability tests performed after autoxidation for one month induced by high temperature are resumed in Table 3.

**Table 3**

| Antioxidant | Temperature (°C) | % w/w Antioxidant | Recovery EPA (%) | Recovery DHA (%) |
|---|---|---|---|---|
| Not used | 60 | - | 52 | 45 |
| Resveratrol | 60 | 1.14 | 68 | 64 |
| Coenzyme Q10 | 60 | 4.32 | 64 | 65 |
| Ascorbyl palmitate | 60 | 2.07 | 58 | 56 |
| α-tocopherol | 60 | 2.15 | 57 | 55 |
| Ascorbic acid | 60 | 0.88 | 56 | 54 |
| Lipoic acid | 60 | 1.00 | 52 | 50 |
| Hydroxytyrosol | 60 | 0.77 | 40 | 36 |
| Oleorupine | 60 | 2,70 | 38 | 35 |
| Epigallocatechin | 60 | 2.30 | 35 | 30 |

After these preliminary stability tests other formulations were prepared using coenzyme Q10, α-tocopherol and resveratrol as antioxidants.

### Formulation 2

| | |
|---|---|
| - n-3 PUFA: | 4,9 g |
| - α-Tocopherol: | 0,1 g |
| - Neusilin U.S.-2: | 5,0 g |

α-Tocopherol was added to n-3 PUFA and the solution was left under mechanical stirring at 600 rpm for 3 hours at 22°C at 60% RH; a clear solution was observed. The solution was then added to Neusilin U.S.-2 in small portions, manually mixing up to obtain a homogeneous solid. 3 month stability tests were carried out at 25 °C, 30 °C and 40 °C as described above. The results obtained are summarized in Table 4.

### Formulation 3

| | |
|---|---|
| - n-3 PUFA: | 4,9 g |
| - Coenzyme Q10: | 0,1 g |
| - Neusilin U.S.-2: | 5,0 g |

Coenzyme Q₁₀ was added to n-3 PUFA and the solution was left under mechanical stirring at 600 rpm for 1 hour at 22°C at 60% RH; a clear solution was observed. The solution was added to Neusilin U.S.-2 in small portions, manually mixing up to obtain a homogeneous solid.
3 month stability tests were carried out at 25°C, 30°C and 40°C as described above.

The results obtained are summarized in Table 4

### Formulation 4

| | |
|---|---|
| - PUFA | 4,9 g |
| - Resveratrol | 0,1 g |
| - Neusilin U.S.-2 | 5,0g |

n-3 PUFA were added to Neusilin U.S.-2 small portions, manually mixing up to obtain a homogeneous solid.

Resveratrol was then added and the formulation was again mixed up to obtain a homogeneous solid.

3 month stability tests were carried out at 25 °C, 30 °C and 40 °C as described above. The results obtained are summarized in Table 4.

**Table 4**

| Formulation | Antioxidant | Temperature (°C) | Recovery EPA (%) | Recovery DHA (%) |
|---|---|---|---|---|
| 2 | α-Tocopherol | 25 | 92 | 89 |
| | | 30 | 80 | 77 |
| | | 40 | 47 | 42 |
| 3 | Coenzyme Q10 | 25 | 0 | 0 |
| | | 30 | 0 | 0 |
| | | 40 | 0 | 0 |
| 4 | Resveratrol | 25 | 96 | 96 |
| | | 30 | 96 | 95 |
| | | 40 | 90 | 89 |

In order to assess the relationship between the amount of resveratrol and the antioxidant efficacy, another formulation was prepared.

### Formulation 5

| | |
|---|---|
| - PUFA: | 4.9 gr |
| - Resveratrol: | 0.12 gr |
| - Neusilin U.S.-2: | 5 gr |

n-3 PUFA were added to Neusilin U.S.-2 in small portions, mixing up to obtain a homogeneous solid.

Resveratrol was then added and the formulation was again mixed up to obtain a homogeneous solid. 3 and 6 month stability tests were carried out at 25°C as described above.

The results obtained are summarized in Table 5.

**Table 5**

| Time (months) | Temperature (°C) | Recovery EPA (%) | Recovery DHA (%) |
|---|---|---|---|
| 3 | 25 | 99 | 95 |
| 6 | 25 | 98 | 94 |

Another inert solid support was also tested.

### Formulation 6

| | |
|---|---|
| - PUFA: | 1.0 g |
| - Syloid XDP: | 1.0 g |

n-3 PUFA were added to Syloid XDP in small portions, mixing up to obtain a homogeneous solid. The results of the stability tests performed after autoxidation induced by radiation and by high temperature are reported in Table 6.

Even in this case a formulation containing resveratrol was prepared, in order to evaluate its stabilizing effect.

### Formulation 7

| | |
|---|---|
| - PUFA: | 0.5 g |
| - Resveratrol: | 0.01 g |
| - Syloid XDP: | 0.5 g |

n-3 PUFA were added to Syloid XDP in small portions, mixing up to obtain a homogeneous solid. Resveratrol was then added and the formulation was again mixed up to obtain a homogeneous solid.

The results of the stability tests performed after autoxidation induced by radiation and by high temperature are resumed in Table 6

**Table 6**

| Formulation | Antioxidant | Temperature (°C) | Time | Recovery EPA (%) | Recovery DHA (%) |
|---|---|---|---|---|---|
| 6 | None | 22 | 24 hours | <5 | <5 |
| | | 60 | 1 month | 67 | 62 |
| 7 | Resveratrol | 22 | 24 hours | 77 | 75 |
| | | 60 | 1 month | 74 | 70 |

### Discussion of the results:

From the data reported in the present invention it is shown that resveratrol is the natural antioxidant that shows the best stabilizing effect on the degradation of omega-3 in solid formulations. In fact, while from the results reported in Table 1 it is possible to observe that already after three months EPA and DHA are significantly degraded, from the initial tests it is evident that resveratrol, Coenzyme Q10, α-tocopherol, and ascorbyl palmitate are able to improve the stability.

However, as demonstrated by the data of Table 4, surprisingly resveratrol is the only natural antioxidant able to maintain stable solid formulations containing omega-3 for longer periods; in fact, the average recovery of omega-3 in the formulations containing 1% of resveratrol is 90% after 3 months at 40°C, while for the formulations containing 1% of α-tocopherol the recovery is only 45%: the antioxidant power of resveratrol can then considered twice with respect of that of α-tocopherol, and this difference is statistically significant

A further advantage is represented by a direct proportionality between the antioxidant efficacy and the amount of resveratrol used: from the data of Table 5 it is possible to note that when the amount of resveratrol is increased from 1% to 1.2% of the recovery of n-3 PUFA is maintained at 96% also after 6 months.

Furthermore, the process of preparation of the present compositions is simple and particularly suitable for industrial applicability.

## Claims

1. A solid composition comprising omega-3 polyunsaturated fatty acids (n-3 PUFA) and resveratrol adsorbed on an inert substrate selected from the group consisting of: fine ultra-light granule of magnesium aluminometasilicate and a silica carrier; wherein the recovery of n-3 PUFA after 6 months at 25°C is at least 96% by weight.

2. The composition according to claim 1, **characterized in that** the recovery of n-3 PUFA after 3 months at 30°C is at least 95% by weight.

3. The composition according to claim 1, **characterized in that** the recovery of n-3 PUFA after 3 months at 40°C is at least 90% by weight.

4. The composition according to claim 1, **characterized in that** the omega-3 polyunsaturated fatty acids (n-3 PUFA) are selected from the group consisting of eicosapentaenoic acid, the docosahexaenoic acid or mixtures thereof.

5. The composition according claim 1, **characterized in that** the ratio between eicosapentaenoic acid and docosahexaenoic acid is comprised between 0.5 and 2.

6. The composition according to claim 1, in which the omega-3 polyunsaturated fatty acids (n-3 PUFA) are in an amount comprised between 0.5 and 1.0 g, preferably between 0.8 and 0.9 g, more preferably 0.9 g.

7. The composition according to claim 1, **characterized in that** the omega-3 polyunsaturated fatty acids (n-3 PUFA) are a mixture of fatty acids having a content in EPA and DHA comprised between 75% and 95% by weight, preferably at least 85%, on the total fatty acids weight, and wherein the total content of n-3 PUFA is at least 90% by weight on the total fatty acids weight.

8. The composition according to claim 1, wherein the omega-3 polyunsaturated fatty acids (n-3 PUFA) are a mixture of ethyl esters of EPA and DHA in a ratio comprised between 0.9 and 1.5 and the content of EPA ethyl ester is comprised between 40 and 51% by weight and the content of DHA ethyl ester is comprised between 34 and 45% by weight on the total fatty acids weight.

9. The composition according to claim 1, **characterized in that** the alkyl ester of omega-3 polyunsaturated fatty acids (n-3 PUFA) are selected from the group consisting of ethyl, methyl, propyl or mixtures thereof.

10. The composition according to claim 1, wherein the ratio between omega-3 polyunsaturated fatty acids (n-3 PUFA) acid and resveratrol is comprised between 0.01 and 0.1, preferred is 0.02.

11. The composition according to claim 1, in which the inert substrate is Neusilin® U.S.-2.

12. The composition according to claims 1-11, in the form of dietary supplement or medicament for oral administration.

13. The composition according to claims 1-12, further comprising one or more vitamins, minerals, coenzymes, antioxidants and/or plant extracts, and/or at least one pharmaceutically acceptable vehicle or excipient

14. The composition according to claims 1-13, **characterized in that** is encapsulated by soft gelatin capsules, optionally having an enteric coating, for oral administration.

15. The composition according to claims 1-14 , for use in preventing or treating cardiovascular diseases due to lipid metabolism disorders and increased platelets aggregation; damages due to free radicals selected from the group consisting of atherosclerosis, cancer, inflammatory joint disease, asthma, diabetes, senile dementia and degenerative eye disease; and/or viral diseases.

## Patentansprüche

1. Feste Zusammensetzung, umfassend mehrfach ungesättigte Omega-3-Fettsäuren (n-3-PUFA) und Resveratrol adsorbiert an einem inerten Substrat, das aus der Gruppe, bestehend aus: feinen ultraleichten Körnern von Magnesiumaluminiumsilicat und einem Siliciumdioxidträger, ausgewählt ist; wobei die Wiedergewinnung von n-3-PUFA nach 6 Monaten bei 25°C wenigstens 96 Gew.-% ist.

2. Zusammensetzung nach Anspruch 1, **dadurch gekennzeichnet, dass** die Wiedergewinnung von n-3-PUFA nach 3 Monaten bei 30°C wenigstens 95 Gew.-% ist.

3. Zusammensetzung gemäß Anspruch 1, **dadurch gekennzeichnet, dass** die Wiedergewinnung von n-3-PUFA nach 3 Monaten bei 40°C mindestens 90 Gew.-% ist.

4. Zusammensetzung gemäß Anspruch 1, **dadurch gekennzeichnet, dass** die mehrfach ungesättigten Omega-3-Fettsäuren (n-3-PUFA) aus der Gruppe, bestehend aus Eicosapentaensäure, Docosahexaensäure oder Gemischen davon, ausgewählt sind.

5. Zusammensetzung gemäß Anspruch 1, **dadurch gekennzeichnet, dass** das Verhältnis zwischen Eicosapentaensäure und Docosahexaensäure zwischen 0,5 und 2 liegt.

6. Zusammensetzung gemäß Anspruch 1, in der die mehrfach ungesättigten Omega-3-Fettsäuren (n-3-PUFA) in einer Menge sind, die zwischen 0,5 und 1,0 g, vorzugsweise zwischen 0,8 und 0,9 g, liegt, bevorzugter 0,9 g ist.

7. Zusammensetzung gemäß Anspruch 1, **dadurch gekennzeichnet, dass** die mehrfach ungesättigten Omega-3-Fettsäuren (n-3-PUFA) ein Gemisch aus Fettsäuren sind, welches einen Gehalt an EPA und DHA hat, der zwischen 75 und 95 Gew.-%, vorzugsweise bei 85 Gew.-%, bezogen auf das Gesamtfettsäuregewicht, liegt und in der der Gehalt an n-3-PUFA wenigstens 90 Gew.-%, bezogen auf das Gesamtfettsäuregewicht, ist.

8. Zusammensetzung gemäß Anspruch 1, wobei die mehrfach ungesättigten Omega-3-Fettsäuren (n-3-PUFA) ein Gemisch von Ethylestern von EPA und DHA in einem Verhältnis, das zwischen 0,9 und 1,5 liegt, sind und der Gehalt an EPA-Ethylester zwischen 40 und 51 Gew.-% liegt und der Gehalt an DHA-Ethylester zwischen 34 und 45 Gew.-%, bezogen auf das Gesamtfettsäuregewicht, liegt.

9. Zusammensetzung gemäß Anspruch 1, **dadurch gekennzeichnet, dass** der Alkylester von mehrfach ungesättigten Omega-3-Fettsäuren (n-3-PUFA) aus der Gruppe, bestehend aus Ethyl, Methyl, Propyl oder Gemischen davon, ausgewählt ist.

10. Zusammensetzung gemäß Anspruch 1, wobei das Verhältnis zwischen mehrfach ungesättigten Omega-3-Fettsäuren (n-3-PUFA) und Resveratrol zwischen 0,01 und 0,1 liegt, bevorzugt 0,02 ist.

11. Zusammensetzung gemäß Anspruch 1, in der das inerte Substrat Neusilin® U.S.-2 ist.

12. Zusammensetzung gemäß den Ansprüchen 1-11 in der Form eines Nahrungsergänzungsmittels oder eines Medikaments zur oralen Verabreichung.

13. Zusammensetzung gemäß den Ansprüchen 1-12, umfassend ein(en) oder mehrere Vitamin(e), Mineralstoff(e), Coenzym(e), Antioxidans/Antioxidantien und/oder Pflanzenextrakt(e) und/oder wenigstens ein pharmazeutisch annehmbares Vehikel oder Exzipiens.

14. Zusammensetzung gemäß den Ansprüchen 1-13, **dadurch gekennzeichnet, dass** sie von weichen Gelatinekapseln, die gegebenenfalls eine enterische Beschichtung haben, zur oralen Verabreichung eingekapselt ist.

15. Zusammensetzung gemäß den Ansprüchen 1-14 zur Verwendung bei der Prävention oder Behandlung kardiovaskulärer Erkrankungen infolge von Lipidmetabolismusstörungen und verstärkter Blutplättchenaggregation; Schädigungen durch freie Radikale, ausgewählt aus der Gruppe, bestehend aus Atherosklerose, Krebs, entzündlicher Gelenkserkrankung, Asthma, Diabetes, seniler Demenz und degenerativer Augenerkrankung, und/oder Viruserkrankungen.

## Revendications

1. Composition solide comprenant des acides gras polyinsaturés oméga-3 (n-3 PUFA) et du resvératrol adsorbée sur un substrat inerte choisi dans le groupe constitué de : granulé fin et ultraléger d'aluminométasilicate de magnésium et support de silice ; dans laquelle la récupération de n-3 PUFA après 6 mois à 25 °C est d'au moins 96 % en poids.

2. Composition selon la revendication 1, **caractérisée en ce que** la récupération de n-3 PUFA après 3 mois à 30 °C est d'au moins 95 % en poids.

3. Composition selon la revendication 1, **caractérisée en ce que** la récupération de n-3 PUFA après 3 mois à 40 °C est d'au moins 90 % en poids.

4. Composition selon la revendication 1, **caractérisée en ce que** les acides gras polyinsaturés oméga-3 (n-3 PUFA) sont choisis dans le groupe constitué de l'acide éicosapentaénoïque, de l'acide docosahexaénoïque ou des mélanges de ceux-ci.

5. Composition selon la revendication 1, **caractérisée en ce que** le rapport entre l'acide éicosapentaénoïque et l'acide docosahexaénoïque est compris entre 0,5 et 2.

6. Composition selon la revendication 1, dans laquelle les acides gras polyinsaturés oméga-3 (n-3 PUFA) sont en une quantité comprise entre 0,5 et 1,0 g, de préférence entre 0,8 et 0,9 g, plus préférablement de 0,9 g.

7. Composition selon la revendication 1, **caractérisée en ce que** les acides gras polyinsaturés oméga-3 (n-3 PUFA) sont un mélange d'acides gras ayant une teneur en EPA et DHA compris entre 75 % et 95 % en poids, de préférence d'au moins 85 %, par rapport au poids total des acides gras, et dans laquelle la teneur totale en n-3 PUFA est d'au moins 90 % en poids par rapport au poids total des acides gras.

8. Composition selon la revendication 1, dans laquelle les acides gras polyinsaturés oméga-3 (n-3 PUFA) sont un mélange d'esters éthyliques de EPA et de DHA dans un rapport compris entre 0,9 et 1,5 et la teneur en ester éthylique de EPA est comprise entre 40 et 51 % en poids et la teneur en ester éthylique de DHA est comprise entre 34 et 45 % en poids par rapport au poids total des acides gras.

9. Composition selon la revendication 1, **caractérisée en ce que** les esters alkyliques d'acides gras polyinsaturés oméga-3 (n-3 PUFA) sont choisis dans le groupe constitué de l'éthyle, du méthyle, du propyle ou des mélanges de ceux-ci.

10. Composition selon la revendication 1, dans laquelle le rapport entre l'acide des acides gras polyinsaturés oméga-3 (n-3 PUFA) et le resvératrol est compris entre 0,01 et 0,1, le rapport étant de préférence de 0,02.

11. Composition selon la revendication 1, dans laquelle le substrat inerte est la Neusilin® U.S.-2.

12. Composition selon les revendications 1 à 11, sous la forme d'un supplément diététique ou d'un médicament pour administration orale.

13. Composition selon les revendications 1 à 12, comprenant en outre un(e) ou plusieurs vitamines, minéraux, coenzymes, antioxydants et/ou extraits de plante, et/ou au moins un véhicule ou excipient pharmaceutiquement acceptable.

14. Composition selon les revendications 1 à 13, **caractérisée en ce qu'**elle est encapsulée par des capsules de gélatine molle, ayant facultativement un enrobage entérique, pour administration orale.

15. Composition selon les revendications 1 à 14, pour utilisation dans la prévention ou le traitement de maladies cardiovasculaires dues à des troubles du métabolisme des lipides et à une agrégation accrue des plaquettes ; de dommages dus aux radicaux libres choisis dans le groupe constitué de l'athérosclérose, du cancer, d'une maladie articulaire inflammatoire, de l'asthme, du diabète, de la démence sénile et d'une maladie oculaire dégénérative ; et/ou de maladies virales.
